# EUROPEAN PATENT APPLICATION

(11) **EP 3 195 863 A1**
(43) Date of publication of application: **26.07.2017**
(21) Application number: 16198852.2
(22) Date of filing: 30.11.2011
(51) Int. Cl.: A61K 31/381, A61K 31/343, A61K 9/00, A61K 8/42, A61K 8/49, A61K 31/167, A61K 31/4015, A61K 31/421, A61K 31/519, A61P 17/02, A61Q 19/08, A61K 8/37, A61K 31/22

(54) **COMPOUNDS AND METHODS FOR SKIN REPAIR**

(30) Priority: 02.12.2010 US 419115 P
(62) Divisional of application: 11794357.1
(71) Applicant: ALLERGAN, INC., Irvine, CA 92612 (US)
(72) Inventor: BURK, Robert M., Laguna Beach, CA California 92651 (US); IM, Wha Bin, Irvine, CA California 92612 (US); WHITCUP, Scott M., Laguna Hills, CA California 92653 (US)
(74) Representative: Hoffmann Eitle

(57) **Abstract**

The disclosure provides compositions and methods for treating a skin blemish. The compositions comprise a therapeutically effective amount of a compound useful for treating skin blemishes such as wounds, scars and wrinkles.

## Description

### CROSS REFERENCE TO RELATED APPLICATIONS

This application claims the benefit of U.S. Provisional Application Serial No. 61/419,115 filed December 2, 2010, which is hereby incorporated by reference in its entirety.

### FIELD OF THE INVENTION

The invention relates generally to compositions and methods for wound healing, and particularly to the use of EP4 agonists for treatment in wound healing, scar reduction, and skin repair.

### BACKGROUND OF THE INVENTION

Prostanoid EP4 receptor is a G protein-coupled receptor that mediates the actions of prostaglandin E2 (PGE2) and is characterized by the longest intracellular C terminus loop when compared to other prostanoid receptors. EP4 receptors couple not only to Gs and mediate elevations in cAMP concentration, but also to Gi and phosphorylate key intracellular signaling proteins such as ERK or AKT . EP2 is another PGE2 receptor subtype analogous to EP4. There are some redundancies in function between EP2 and EP4 receptors. For example, both receptors induce IOP lowering, and are involved in PGE2-mediated RANKL through cAMP signalings. There are some functional differences, however, primarily arising from differences in receptor density at the target sites: Thus, EP2 is involved in cumulus expansion in ovulation and fertilization, and EP4 regulates closure of the ductus arteriosus. Expression of EP4 receptors is controlled by various physiological and pathophysiological processes as these receptors participate in ovulation and fertilization, induce bone formation, protect against inflammatory bowel disease, facilitate Langerhans cell migration and maturation and mediate joint inflammation in a model of collagen-induced arthritis, among others

Skin blemishes such as flesh wounds, scars from cosmetic surgery including, but not limited to, breast implants, from surgeries on the back, central chest, heart, abdomen, pubic area, and joint, from burns, aging, and photoaging, and wrinkles can occur on any area of the body. Scarring may occur in all parts of adult body, following local or systemic traumas such as mechanical injury, surgery, burn, chemical contact, radiation and poisoning, and represents a failure of homeostatic processes to restore normal structure at the wound sites. Wrinkles occur for a variety of reasons and are a common sign of aging. Both scars and signs of aging can typically considered undesirable. Moreover, the prevention of unsightly scars from any injuries or in people at high risk of bad scars is highly desirable.

Accordingly, an agent that safely and effectively treats or prevents such skin blemishes is highly desirable.

### SUMMARY OF THE INVENTION

The disclosure provides compositions and methods for wound healing and scar reduction. The compositions and methods of the invention include at least one EP4 agonist set forth herein. Wounds, scars, and other skin blemishes that can be treated or prevented by the compositions and methods of the invention can arise from events such as surgery on all parts of the body, trauma, disease, mechanical injury, burn, radiation, poisoning, photoaging, aging, chemical contact and the like.

In one embodiment of the invention, there are provided methods for treating skin blemishes. Such methods can be performed, for example, by administering to a subject in need thereof a therapeutically effective amount of at least one EP4 agonist or EP4/EP2 dual agonist, or the combination of EP4 and EP2 agonists, thereby treating the skin blemish.

In one embodiment, a method is provided for healing a wound that includes administering to a subject in need thereof a composition comprising a therapeutically effective amount of a compound having a structure: 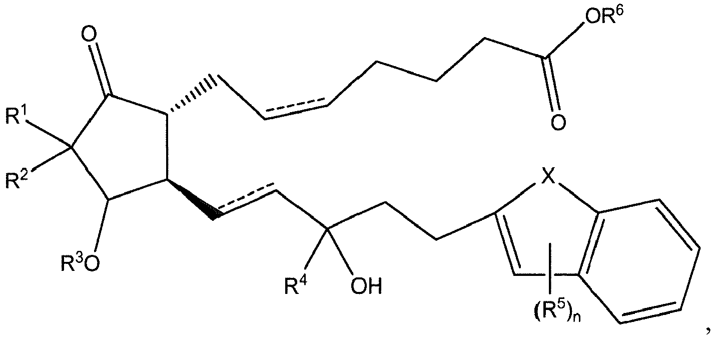 wherein each dashed line represents the presence or absence of a double bond; R¹, R¹, R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

In another embodiment, a method is provided for treating a flesh wound that comprises administering a composition comprising a therapeutically effective amount of a compound having a structure: 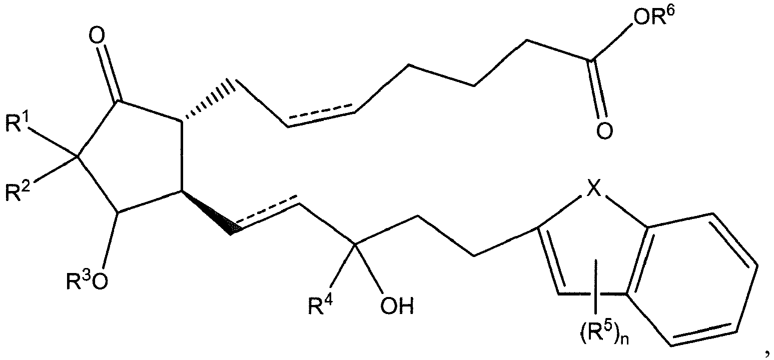 wherein each dashed line represents the presence or absence of a double bond; R¹, R¹, R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
wherein the wound heals more normally than without administration of the composition.

In yet another embodiment, a method of reducing the appearance of a wrinkle comprising administering to said wrinkle a composition comprising a therapeutically effective amount of a compound having a structure: 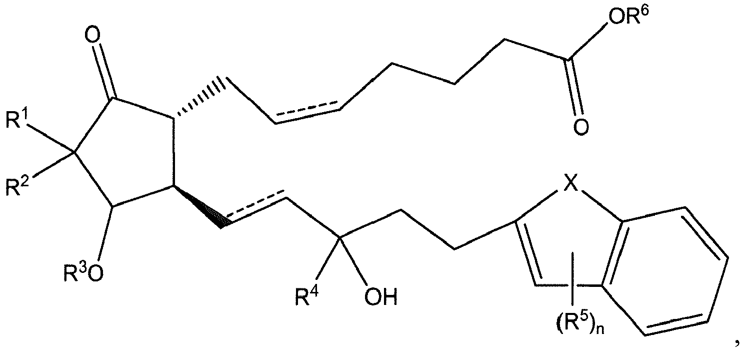 wherein each dashed line represents the presence or absence of a double bond; R¹, R¹, R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
wherein the appearance of the wrinkle is diminished.

In another embodiment, the compositions of the invention include at least one EP4 agonist having the structure: 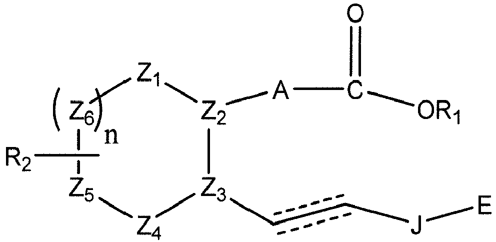 wherein:
each of Z₁ to Z₆ is independently C, N, O, or S;
A is -(CH₂)₆-, or *cis* -CH₂CH=CH-(CH₂)₃-, wherein 1 or 2 carbons may be substituted with S or O; or
A is -(CH₂)ₘ-Ar-(CH₂)ₒ- wherein Ar is arylene or heteroarylene, the sum of m and o is from 1 to 4, and wherein one CH₂ may be substituted with S or O;
R₁ is H, alkyl, cycloalkyl, oxyalkyl, hydroxyalkyl, alkenyl, oxyalkenyl, or hydroxyalkenyl;
R₂ is alkyl, hydroxyl, halide, or oxo;
J is alkylene, cycloalkylene, oxyalkylene, hydroxyalkylene, fluoroalkylene, difluoroalkylene;
E is C₁₋₁₂ alkyl, R₃, or -Y-R₃ wherein Y is CH₂, S, or O, and R₃ is aryl or heteroaryl;
n is 0 or 1;
and wherein a dashed line represents the presence or absence of a bond.

### BRIEF DESCRIPTION OF THE DRAWINGS

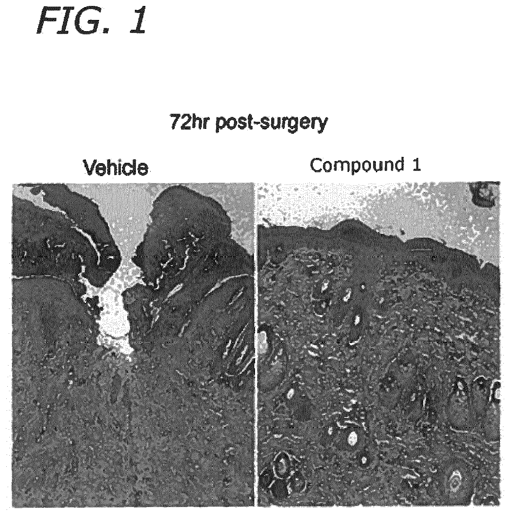

**Figure 1** is an image of a hematoxylin & eosin (H&E) stained skin biopsy samples at 3 days post skin incisional surgery. The image shows epidermal coverage of the wound site (magnification 200x).

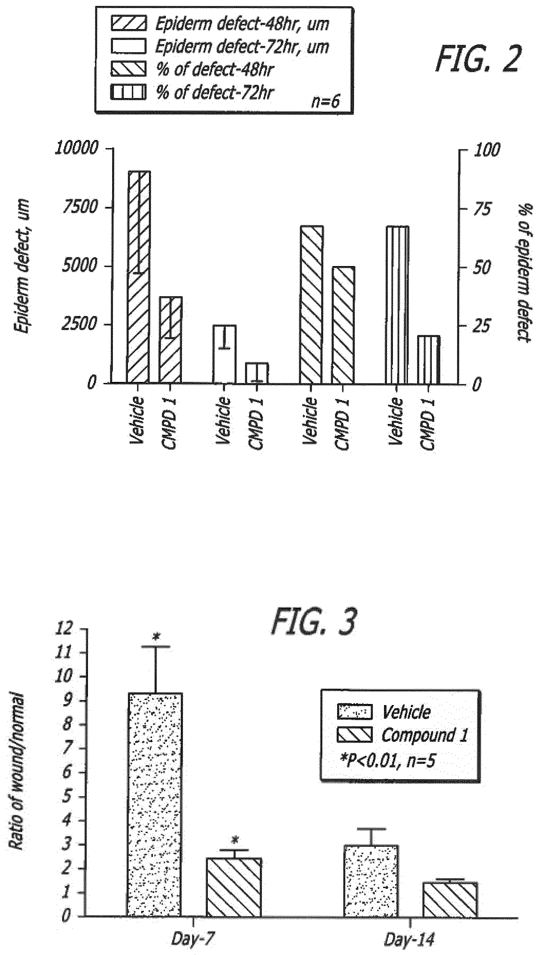

**Figure 2** is a graph showing an epidermal defect (µm and percentage) at 2 and 3 days post-surgery for vehicle treated and Compound 1 treated groups.
**Figure 3** is a graph showing epidermal thickness at wound sites compared to nearby normal sites (ratio wound/normal) at 7 and 14 days post-surgery in groups treated with vehicle and Compound 1.

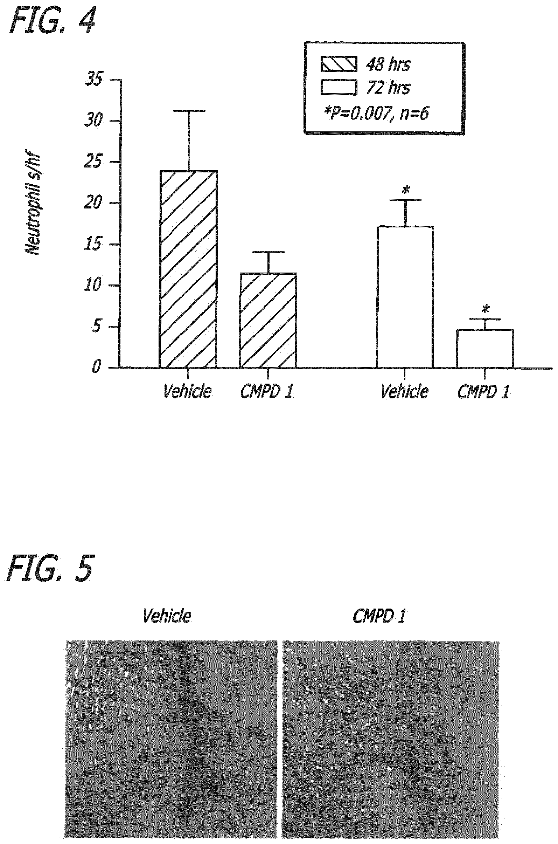

**Figure 4** is a graph showing quantification of neutrophils (s/hf) at wound sites at 2 and 3 days post-surgery in groups treated with vehicle and Compound 1. Neutrophils at the dermis region were counted under 400x magnification.
**Figure 5** is an image showing macroscopic appearances of skin wound sites at 14 days post-surgery in vehicle treated and Compound 1 treated skin at a magnification of 6.5x.

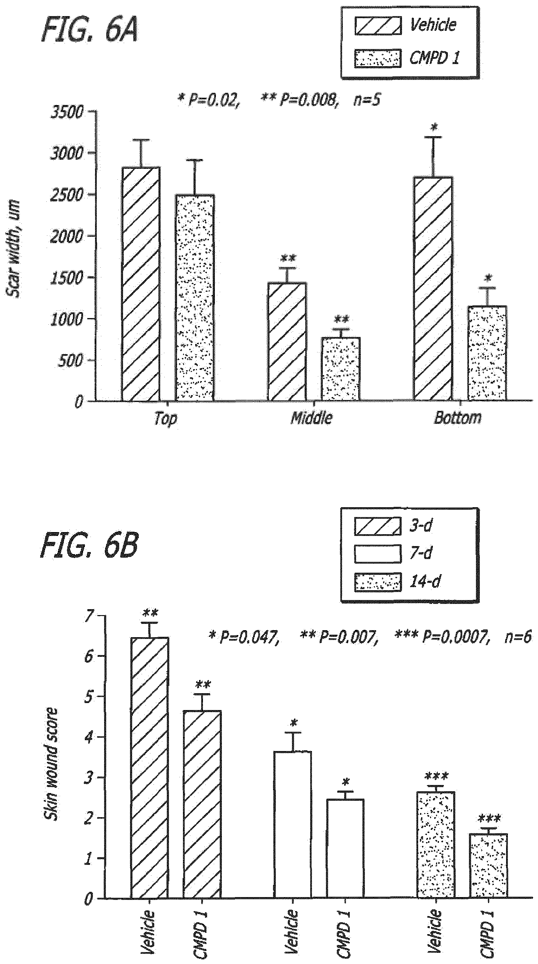

**Figures 6A** and **B** are graphs quantifying skin scar tissue sections and gross tissue appearance of samples treated with either vehicle or Compound 1. **Figure 6A** shows scar width (µm) on Masson trichrome stained sections at the top, middle and bottom of the section. **Figure 6B** shows the gross skin wound score at days 3, 7, and 14 post-surgery.

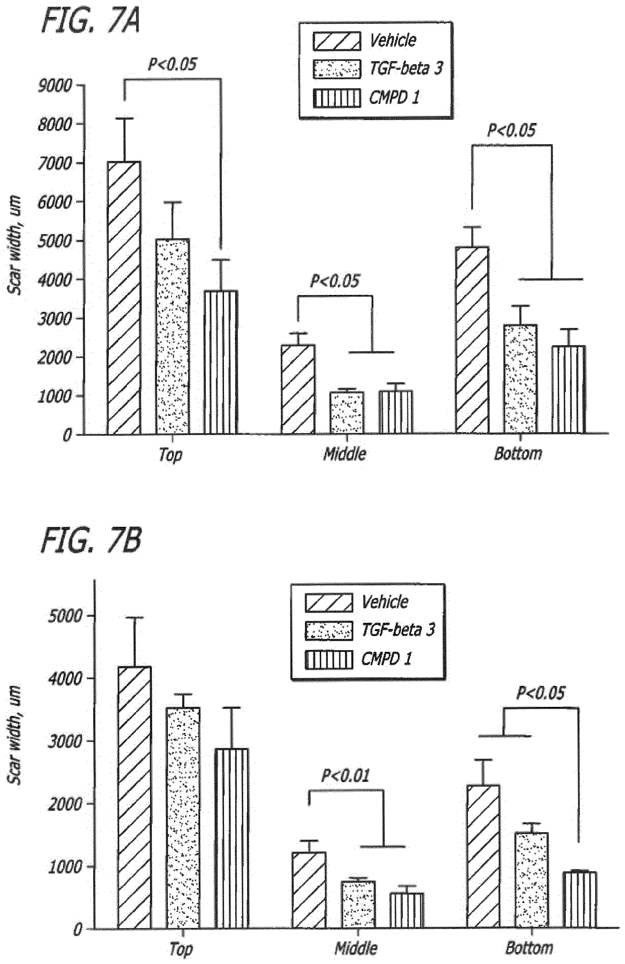

**Figures 7A** and **B** are graphs quantifying skin scar width on wound sections at 2 weeks post-surgery. **Figure 7A** shows scar width (µm) of picrosirius red stained sections in the top, middle and bottom. **Figure 7B** shows scar width at the top, middle and bottom sections of Masson trichrome stained sections treated with either vehicle, TGF-β3, or Compound 1.

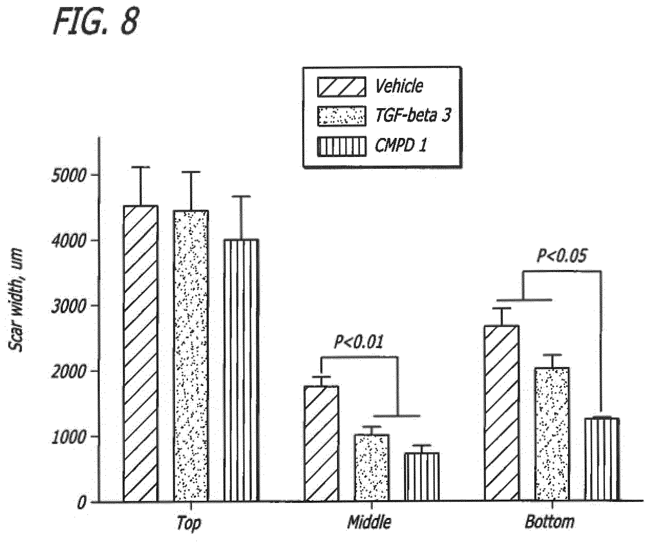

**Figure 8** is a graph quantifying skin scar width based on Masson trichrome staining 70 days post-surgery in tissue treated with vehicle, TGF-β3, or Compound 1.

### DETAILED DESCRIPTION OF THE INVENTION

It is to be understood that both the foregoing general description and the following detailed description are exemplary and explanatory only and are not restrictive of the invention claimed. As used herein, the use of the singular includes the plural unless specifically stated otherwise. As used herein, "or" means "and/or" unless stated otherwise. Furthermore, use of the term "including" as well as other forms, such as "includes," and "included," is not limiting. The section headings used herein are for organizational purposes only and are not to be construed as limiting the subject matter described.

Unless specific definitions are provided, the nomenclatures utilized in connection with, and the laboratory procedures and techniques of analytical chemistry, synthetic organic and inorganic chemistry described herein are those known in the art. Standard chemical symbols are used interchangeably with the full names represented by such symbols. Thus, for example, the terms "hydrogen" and "H" are understood to have identical meaning. Standard techniques may be used for chemical syntheses, chemical analyses, and formulation.

As used herein, "alkyl" refers to straight or branched chain hydrocarbyl groups having from 1 up to about 100 carbon atoms. Whenever it appears herein, a numerical range, such as "1 to 100" or "C₁-C₁₀₀", refers to each integer in the given range; *e.g*., "C₁-C1₀₀ alkyl" means that an alkyl group may comprise only 1 carbon atom, 2 carbon atoms, 3 carbon atoms, *etc*., up to and including 100 carbon atoms, although the term "alkyl" also includes instances where no numerical range of carbon atoms is designated. "Substituted alkyl" refers to alkyl moieties bearing substituents including alkyl, alkenyl, alkynyl, hydroxy, oxo, alkoxy, mercapto, cycloalkyl, substituted cycloalkyl, heterocyclic, substituted heterocyclic, aryl, substituted aryl, heteroaryl, substituted heteroaryl, aryloxy, substituted aryloxy, halogen, haloalkyl, cyano, nitro, nitrone, amino, lower alkylamino, lower alkyldiamino, amido, azido, -C(O)H, -C(O)R₇, -CH₂OR₇, -C(O)-, -C(O)-, -S-, -S(O)₂, -OC(O)-O-, wherein R₇ is H or lower alkyl, acyl, oxyacyl, carboxyl, carbamate, sulfonyl, sulfonamide, sulfuryl, and the like. As used herein, "lower alkyl" refers to alkyl moieties having from 1 to about 6 carbon atoms.

As used herein, "alkenyl" refers to straight or branched chain hydrocarbyl groups having at least one carbon-carbon double bond, and having in the range of about 2 up to about 100 carbon atoms, and "substituted alkenyl" refers to alkenyl groups further bearing one or more substituents as set forth above. As used herein, "lower alkenyl" refers to alkenyl moieties having from 2 to about 6 carbon atoms.

As used herein, "alkynyl" refers to straight or branched chain hydrocarbyl groups having at least one carbon-carbon triple bond, and having in the range of about 2 up to about 100 carbon atoms, and "substituted alkynyl" refers to alkynyl groups further bearing one or more substituents as set forth above. As used herein, "lower alkynyl" refers to alkynyl moieties having from 2 to about 6 carbon atoms.

As used herein, "cycloalkyl" refers to cyclic (i.e., ring-containing) alkyl moieties typically containing in the range of about 3 up to about 8 carbon atoms, and "substituted cycloalkyl" refers to cycloalkyl groups further bearing one or more substituents as set forth above.

As used herein, "aryl" refers to aromatic groups having in the range of 5 up to 14 carbon atoms and "substituted aryl" refers to aryl groups further bearing one or more substituents as set forth above.

As used herein, "heteroaryl" refers to aromatic moieties containing one or more heteroatoms (e.g., N, O, S, or the like) as part of the ring structure and having in the range of 5 up to 14 total atoms in the ring structure (i.e., carbon atoms and heteroatoms). "Substituted heterocyclic" refers to heterocyclic groups further bearing one or more substituents as set forth above.

As used herein, "heterocyclic" refers to non-aromatic cyclic (i.e., ring-containing) groups containing one or more heteroatoms (e.g., N, O, S, or the like) as part of the ring structure, and having in the range of 3 up to 14 carbon atoms and "substituted heterocyclic" refers to heterocyclic groups further bearing one or more substituents as set forth above.

As used herein, "halogen" or "halide" refers to fluoride, chloride, bromide or iodide. "Fluoride, chloride, bromide or iodide" may also be referred to as "fluoro, chloro, bromo, or iodo".

As used herein the "ene" suffix when added to the end of a term such as, for example, "alkyl" (resulting in "alkylene") refers to an alkyl moiety which connects to two other parts of a molecule, i.e. the two parts are bonded to the alkyl moiety in two distinct positions. In other words, the alkyl moiety occupies an internal position of the molecule.

Disclosed herein are compositions and methods for wound healing and scar reduction. In one embodiment the compositions described herein comprise compounds having a general structure: 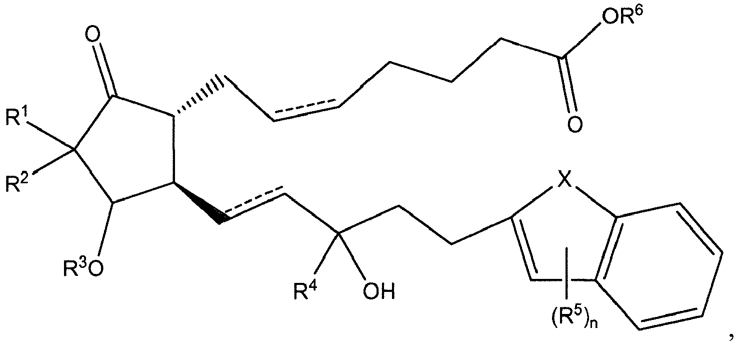 wherein each dashed line represents the presence or absence of a double bond; R¹, R², R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

In certain embodiments, R⁴ is H, R³ is H, and X is S.

In another embodiment, R¹ and R² are CH₃.

In a further embodiment, R⁵ is Cl.

In yet another embodiment, the compound is:

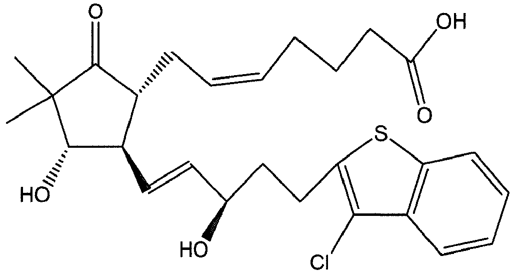

In another embodiment, the methods of the invention employ compositions including at least one EP4 agonist having the structure: 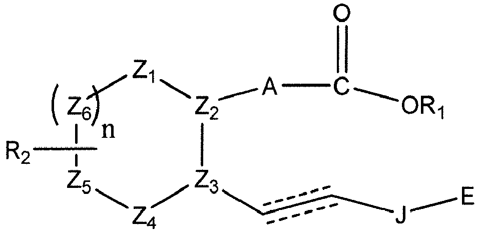 wherein:
each of Z₁ to Z₆ is independently C, N, O, or S;
A is -(CH₂)₆-, or *cis* -CH₂CH=CH-(CH₂)₃-, wherein 1 or 2 carbons may be substituted with S or O; or
A is -(CH₂)ₘ-Ar-(CH₂)ₒ- wherein Ar is arylene or heteroarylene, the sum of m and o is from 1 to 4, and wherein one CH₂ may be substituted with S or O;
R₁ is H, alkyl, cycloalkyl, oxyalkyl, hydroxyalkyl, alkenyl, oxyalkenyl, or hydroxyalkenyl;
R₂ is alkyl, hydroxyl, halide, or oxo;
J is alkylene, cycloalkylene, oxyalkylene, hydroxyalkylene, fluoroalkylene, difluoroalkylene;
E is C₁₋₁₂ alkyl, R₃, or -Y-R₃ wherein Y is CH₂, S, or O, and R₃ is aryl or heteroaryl;
n is 0 or 1;
and wherein a dashed line represents the presence or absence of a bond.

In another embodiment of the invention, a method is provided for treating a skin blemish that comprises administering a composition comprising a therapeutically effective amount of at least one compound having a structure:

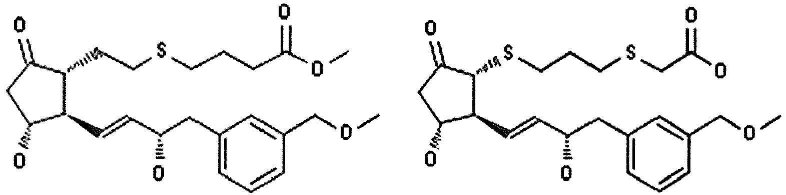

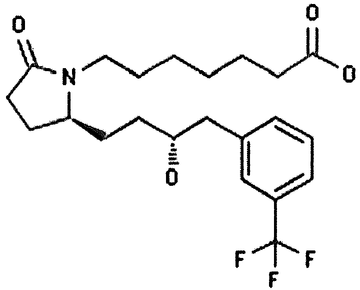

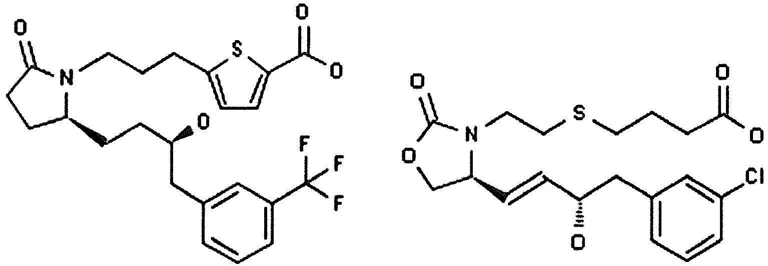

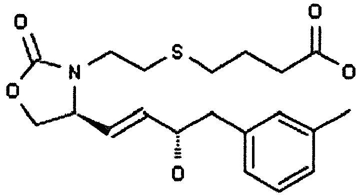

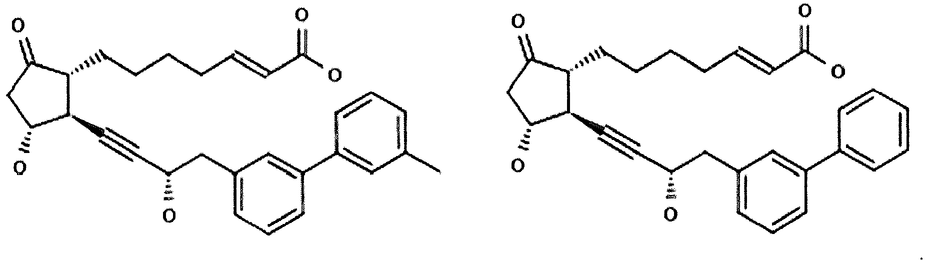

In another embodiment of the invention, a method is provided for treating a skin blemish that comprises administering a composition comprising a therapeutically effective amount of at least one compound having a structure:

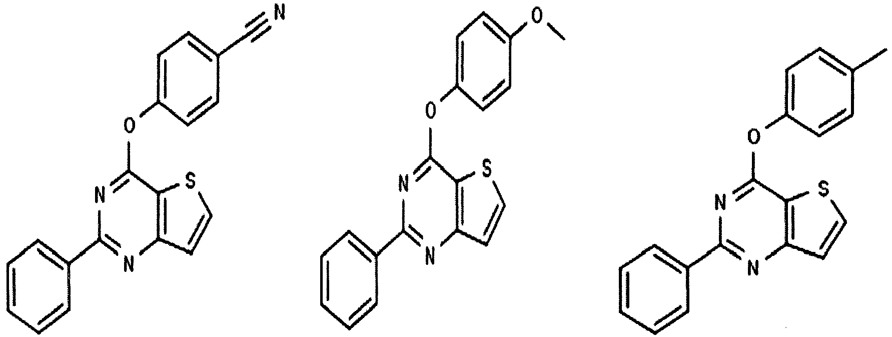

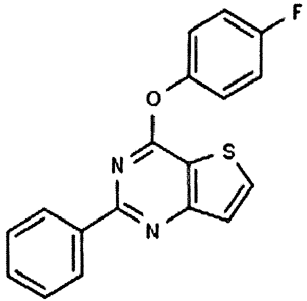

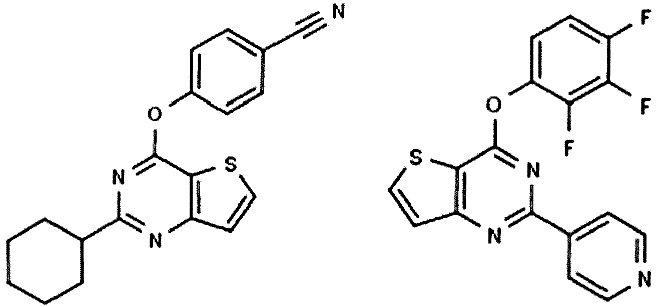

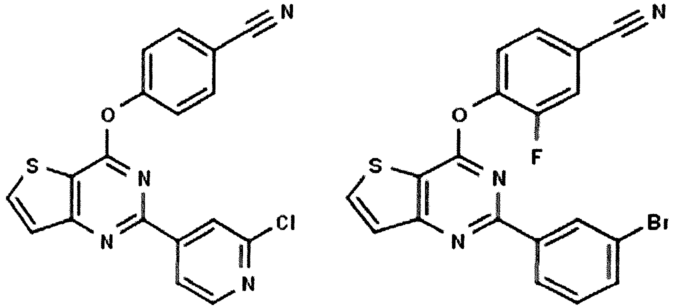

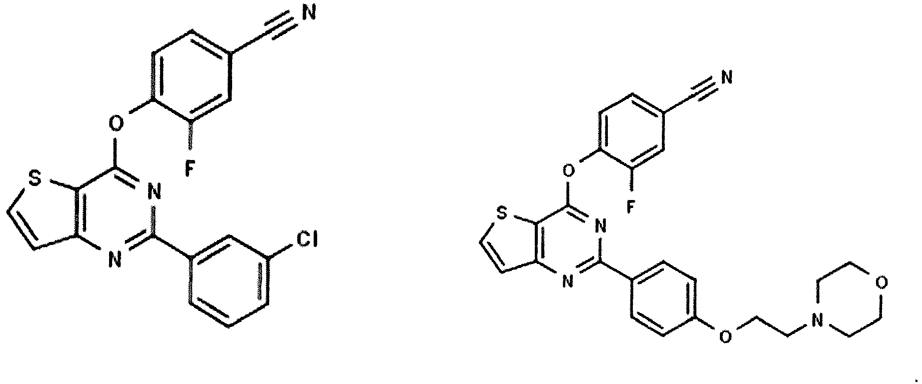

In another embodiment of the invention, a method is provided for treating a skin blemish that comprises administering a composition comprising a therapeutically effective amount of at least one compound having a structure:

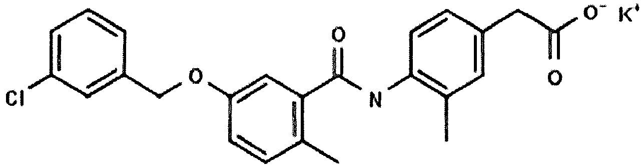

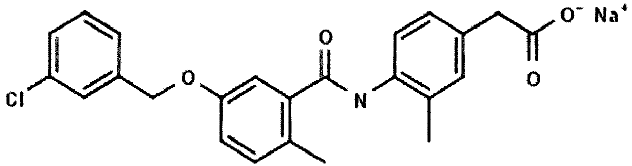

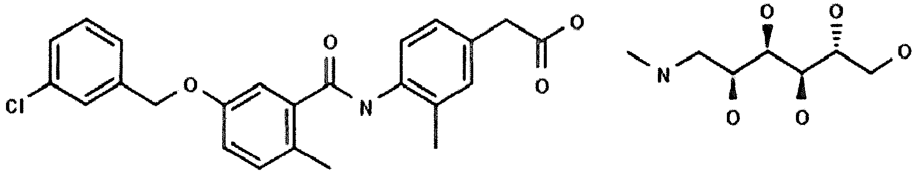

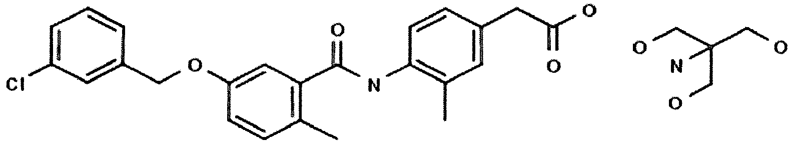

In another embodiment, the methods of the invention employ compositions including at least one EP4 agonist having the structure: 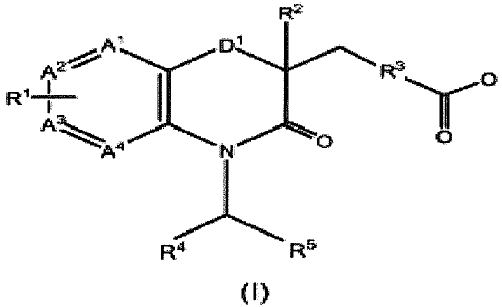 wherein:
A¹, A², A³ and A⁴ are each independently selected from C, N, CR^{A}, NR^{A}, C-(O)R^{A}, N-(O)R^{A}, C-(R^{A}R^{B}) and N-(R^{A}R^{B});
R¹, R¹, R³, R⁴ and R⁵ are each independently selected from a bond, H, halo, cyano, nitro, oxo, CF₃, OCF₃, C1-5 alkyl, C2-8 alkenyl, C2-8 alkynyl, C1-8 hydroxyalkyl, C1-8 haloalkyl, C₃₋₁₀ cycloalkyl, C₄₋₁₀ heterocycloalkyl, C3-10 cycloalkoxy, C1-8 alkoxy, C1-8 alkoxyalkyl, C₅₋₁₀ aryl, and C₅₋₁₀ heteroaryl, wherein each of said C1-8 alkyl, C₂₋₈ alkenyl, C₂₋₈ alkynyl, C1-8 hydroxyalkyl, C1-8 haloalkyl, C₃₋₁₀ cycloalkyl, C₄₋₁₀ heterocycloalkyl, C₃-₁₀ cycloalkoxy, C1-8 alkoxy, C1-8 alkoxyalkyl, C₅₋₁₀ aryl, and C5-10 heteroaryl moieties is optionally substituted by one or more of R^{A}, R^{A}R^{B}, CR^{A}, CR^{A}R^{B}, SR^{A}, SR^{A}R^{B}, OR^{A}, COR^{A}, S(O)ₐR^{A}, NR^{A}R^{B}, CONR^{A}R^{B}, N(O)R^{A}R^{B}, (CR^{A}R^{B})_{b}(C₅₋io aryl), (CR^{A}R^{B})_{b}(C₅₋io heteroaryl) or (CR^{A}R^{B})_{b}(C₃₋₁₀ cycloalkyl);
R^{A} and R^{B} are independently selected from a bond, H, halo, cyano, nitro, oxo, CF₃, OCF₃, C1-8 alkyl, C₂₋₈ alkenyl, C₂₋₈ alkynyl, C₅₋₁₀ aryl, C₅-₁₀ heteroaryl, arylalkyl, C₃-₁₀ cycloalkyl, C₄₋₁₀ heterocycloalkyl, C₃₋₁₀ cycloalkoxy, C1-8 alkoxy, and C₂₋₈ alkoxyalkyl;
D¹ is C, N, O, S, CR^{A}, NR^{A}, OR^{A}, SR^{A}, C-(O)R^{A}, N-(O)R^{A}, C-(O)OR^{A}, N-(O)OR^{A}, C-(R^{A}R^{B}), N-(R^{A}R^{B}), or S-(R^{A}R^{B}); and each a is independently selected from 0, 1, 2, 3, 4 and 5; each b is independently selected from 0, 1, 2, 3, 4 and 5; or a pharmaceutically acceptable salt or stereoisomer thereof.

In another embodiment, the methods of the invention employ compositions including at least one EP4 agonist having the structure: 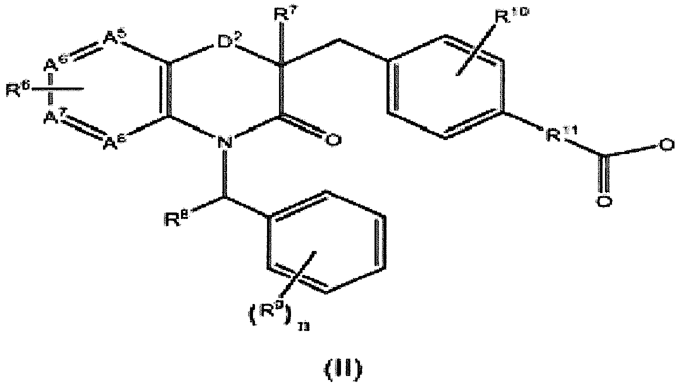 wherein:
A⁵, A⁶, A⁷ and A⁸ are each independently selected from C, N, CR^{C}, NR^{C}, C- (O)R^{C}, N-(O)R⁰, C-(R⁰R⁰) and N-(R⁰R⁰);
R⁶ and R⁷ are each independently selected from a bond, H, halo, cyano, nitro, oxo, CF₃, OCF₃, C1-8 alkyl, C₂₋₈ alkenyl, C₂₋₈ alkynyl, C1-8 hydroxyalkyl, C1-8 haloalkyl, C₃₋₁₀ cycloalkyl, C₄₋₁₀ heterocycloalkyl, C₃₋₁₀ cycloalkoxy, C1-8 alkoxy, C1-8 alkoxyalkyl, C₅₋₁₀ aryl, and C₅₋₁₀ heteroaryl, wherein each of said C1-8 alkyl, C₂₋₈ alkenyl, C₂₋₈ alkynyl, C1-8 hydroxyalkyl, C1-8 haloalkyl, C₃₋₁₀ cycloalkyl, C₄₋₁₀ heterocycloalkyl, C₃₋₁₀ cycloalkoxy, C1-8 alkoxy, C1-8 alkoxyalkyl, C₅₋₁₀ aryl, and C5-10 heteroaryl moieties is optionally substituted by one or more of R^{c}, R^{C}R^{D}, CR^{C}, CR^{C}R^{D}, NR^{C}, OR^{C}, SR^{C}, SR^{C}R^{D}, COR^{C}, S(O)ₙR⁰, NR^{C}R^{D}, CONR⁰R⁰, N(O)R⁰R⁰, (CR⁰R⁰)ₘ(C₅₋₁₀ aryl), (CR⁰R⁰)ₘ(C₅₋₁₀ heteroaryl) or (CR^{c}R^{o})ₘ(C₃₋₁₀ cycloalkyl); R⁸ is H, halo, cyano, nitro, oxo, CF₃, OCF₃, C1-8 alkyl, C₂₋₈ alkenyl, C₂₋₈ alkynyl, C1-5 hydroxyalkyl, C1-8 haloalkyl, C₃₋₁₀ cycloalkyl, C₄₋₁₀ heterocycloalkyl, C₃₋₁₀ cycloalkoxy, C1-5 alkoxy, C1-8 alkoxyalkyl, C5-10 aryl, or C5-10 heteroaryl; R⁹ is H, halo, cyano, nitro, oxo, CF₃, OCF₃, R¹⁰ is H, halo, cyano, nitro, oxo, CF₃, OCF₃, Ci₋₈ alkyl, C₂₋₈ alkenyl, C₂₋₈ alkynyl, Ci₋₈ hydroxyalkyl, Ci₋₈ haloalkyl, C₃₋i₀ cycloalkyl, C₄₋i₀ heterocycloalkyl, C₃₋i₀ cycloalkoxy, Ci₋₈ alkoxy, Ci₋₈ alkoxyalkyl, C₅₋io aryl, or C5-10 heteroaryl;
R¹¹ is a bond, oxo, R°, OR⁰, Ci₋₈ alkyl, C₂₋₈ alkenyl, C₂₋₈ alkynyl, Ci₋₈ hydroxyalkyl, Ci₋₈ haloalkyl, C₃₋i₀ cycloalkyl, C₄₋i₀ heterocycloalkyl, C₃₋i₀ cycloalkoxy, Ci₋₈ alkoxy, Ci₋₈ alkoxyalkyl, C₅₋io aryl, or C₅-₁₀ heteroaryl, wherein each of said Ci₋₈ alkyl, C₂₋₈ alkenyl, C_{2- 8} alkynyl, Ci₋₈ hydroxyalkyl, Ci₋₈ haloalkyl, C₃₋i₀ cycloalkyl, C₄₋i₀ heterocycloalkyl, C₃₋i₀ cycloalkoxy, Ci₋₈ alkoxy, Ci₋₈ alkoxyalkyl, C₅₋io aryl, and C₅₋io heteroaryl moieties is optionally substituted by one or more of CR⁰, CR⁰R⁰, SR⁰, SR⁰R⁰, OR⁰, COR⁰, S(O)ₘR°, NR⁰R⁰, CONR⁰R⁰, N(O)R⁰R⁰, (CR^{c}R⁰)ₘ(C₅-io aryl), (CR^{c}R⁰)ₘ(C₅-io heteroaryl) or (CR^{o}R⁰)ₘ(C₃₋i₀ cycloalkyl);
R^{c} and R^{D} are independently selected from a bond, H, halo, cyano, nitro, oxo, CF₃, OCF₃, Ci₋₈ alkyl, C₂₋8 alkenyl, C₂₋8 alkynyl, C₅₋io aryl, C₅₋io heteroaryl, arylalkyl, C₃₋i₀ cycloalkyl, C₄₋i₀ heterocycloalkyl, C₃₋i₀ cycloalkoxy, Ci₋₈ alkoxy, and C_{2-S} alkoxyalkyl; D² is C, N, O, S, CR^{C}, NR^{C}, OR^{C}, SR^{C}, C-(O)R⁰, N-(O)R⁰, C-(O)OR⁰, N-(O)OR⁰, C-(R⁰R⁰), N-(R⁰R⁰), or S-(R⁰R⁰); and each m is independently selected from O, 1, 2, 3, 4 and 5; and each n is independently selected from O, 1, 2, 3, 4 and 5; or a pharmaceutically acceptable salt or stereoisomer thereof.

In another embodiment, the methods of the invention employ compositions including at least one EP4 agonist having the structure: 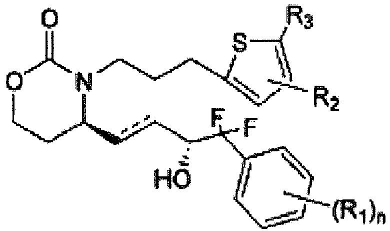 or a pharmaceutically acceptable salt, enantiomer, diastereomer, prodrug or mixture thereof,
wherein,
R and R4 independently represent H, or C1-6 alkyl;
R1 independently represents hydrogen, C1-6 alkyl, halogen, CF3, aryl, said aryl optionally substituted with 1 to 3 groups of halogen, Q -6 alkyl, CF3, or N(R4)2 ;
R2 represents H, or halogen;
R3 represents COOR or carboxylic acid isostere;
n represents 0-3; and
- represents a double or single bond.

In another embodiment, the methods of the invention employ compositions including at least one EP4 agonist having the structure: 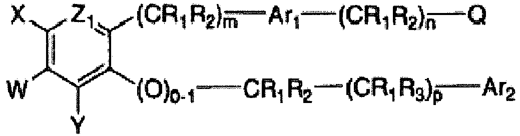 or a pharmaceutically acceptable salt, enantiomer, diastereomer, prodrug or mixture thereof,
wherein,
Z1 represents C-W1, orN;
W, W1 and X independently are H, NR4R4, or halogen;
Y represents hydrogen, halogen, C1-4 alkoxy, C1-4 alkyl, C2-4 alkenyl, aryl, heterocyclyl, C3-6 cycloalkyl, NO2 or CF3, said alkyl, alkenyl, aryl and heterocyclyl optionally substituted with 1-3 groups
R1 and R2 independently are H, halogen, or C1-4 alkyl;
or R1 and 2 may optionally be linked together to form a 3 to 5 membered carbon ring optionally interrupted with 1-2 heteroatoms chosen from O, S, SO, SO2, and N9; R3 represents R1 or OH or R3 and R1 attached to the same carbon may form a carbonyl group;
Q is CO2R4, tetrazolyl, SO3R4, -CF2SO2NH2, -SO2NH2, CONHSO2R5, SO2NHCOR7, - PO(OH)2, CONHP02R6. CONHRd, -COCH2OH, or heterocyclyl containing acidic hydroxyl groups, said heterocyclyl unsubstituted or substituted with 1 to 3 groups of R1O; Ar1 represents phenyl, pyridinyl or thienyl provided that the two substituents (CRτR2)n and (CRτR2)m are para to each other for phenyl and pyridinyl or on the 2,5-positions of the thienyl; said Ari optionally substituted with 1-3 groups of R1O;
Ar2 represents 2,1,3-benzoxadiazol-5-yl, phenyl, pyridyl or thienyl, optionally substituted with 1-3 groups selected from halogen, -6 alkyl, OC1-6 alkyl, CO2H, SC1-6 alkyl, CF3, OCF₃, and SCF3;
R4 represents H or C1-6 alkyl;
R5, R6, R7 and R8 represents C1-6 alkyl, CF3, aryls, heteroaryls, heterocyclyls, Z-Aryl or Z-heteroaryl, said aryls, heteroaryls, heterocyclyls being unsubstituted or substituted with 1 to 3 groups of R1O;
Z is an optional linker containing 0-4 carbon atoms, optionally substituted with C1-4 alkyl; R9 represents hydrogen, C1-6 alkyl, said alkyl optionally substituted with 1-3 halogen, CN, OH, C1-6 alkoxy, C1-4 acyloxy or amino;
R1O represents halogen, C1-6 alkoxy, C1-6 alkyl, CF3, cyano, aryls, heteroaryls, heterocyclyls, SC1-6 alkyl, SC6-10 aryl SC5-10 heterocyclyl, OC6-10 aryl, OC5 0 heterocyclyl, CH2OC1-6 alkyl, CH2SC1-6 alkyl, CH2Saryl;
m represents 2 or 3;
n represents 0 or 1 ; and
p represents 0-2.

Methods of preparing the disclosed compounds and additional compounds suitable for use in the methods disclosed herein, can be found in, e.g., Donde, et el., 10,10-Dialkyl Prostanoic Acid Derivatives as Agents for Lowering Intraocular Pressure, U.S. Patent 6,875,787; Donde, et el., 10,10-Dialkyl Prostanoic Acid Derivatives as Agents for Lowering Intraocular Pressure, U.S. Patent Publication 2004/0235958; Donde, et al., Treatment of Inflammatory Bowel Disease, U.S. Patent Publication 2005/0164992, each of which is hereby incorporated by reference in its entirety.

As used herein, the term "skin blemish" includes a flesh wound, scars from cosmetic surgery including, but not limited to, breast implants, from surgeries on the back, central chest, heart, abdomen, C-section, pubic area, and joint, or from burns, aging and photoaging, or wrinkles can occur on any region of the skin of a body.

A "flesh wound" can be any area in which the structural integrity of the exterior surface of the skin is compromised. A flesh wound can be due to incision, laceration, abrasion, thermal burn, chemical burn, radiation, chemical poisoning or puncture of the skin. The wound can be superficial or extend to the deeper layers of the dermis, subcutaneous, deep fascia, muscle, bone or other internal organs.

A "scar" is an area of abnormal skin appearances arising from fibrous tissue formation (fibrosis) or sclerosis, for example scleroderma, or a loss of normal skin components , after various surgical procedures, injury, burn, irradiation, chemical contact, or diseases including various infections on all parts of the body. Scar types include, but not limited to, hypertrophic scars, recessed scars, and stretch marks. Hypertrophic scars occur when the body overproduces collagen, which causes the scar to be raised above the surrounding skin. An example of a hypertrophic scar is a keloid scar, including the prevention of recurrence of fibrous tissue growth after excision of existing keloid scars. Atrophic, or recessed scars, have a sunken appearance and result when underlying support structure in the skin is lost. Stretch marks (striae) occur when skin is stretched rapidly (i.e., due to significant weight gain or growth spurt or post pregnancy), or when skin is put under tension during the healing process, typically near a joint. As used herein, the term "scar" encompasses any type of scar in the skin due to any cause.

As used herein, the term "wrinkle" is a fold, ridge, crease, furrow, pit, crater, or sunken area in the skin that can be caused by habitual facial expressions, loss of collagen and/or elasticity due to aging, sun damage, smoking, poor hydration, and various other factors. A wrinkle can range from a deep crease to a fine line. Wrinkles occurring on any part of a body, in particular, wrinkles on head or neck of a subject are contemplated herein. Wrinkles that can be treated in accordance with the disclosure include, but are not limited to, a brow furrow, crow's feet, nasolabial fold, one or more lines under the eyes or between the eye brows, and combinations thereof.

As used herein, "treatment" means to prevent and alleviate (or to eliminate) one or more features of a skin blemish either temporarily or permanently. When the compositions are administered to treat a wound, the compositions promote normal healing compared to a wound without the administration. That is, the size (length, depth, height and/or width), character, color and/or texture of the treated wound more closely resemble normal, non-wounded tissue. In this regard, treatment of a wound with the disclosed compositions can prevent, minimize or improve the appearance of a scar formation resulting from healing of the wound. Further, when the disclosed compositions are administered to treat a wrinkle, the wrinkle is treated if the appearance or prominence of the wrinkle is visibly or clinically diminished. That is the length and/or depth is decreased compared to the wrinkle prior to treatment. Alternatively, treatment can comprise prevention of a wrinkle. In this regard, the disclosed compositions can be applied to a region of the skin that typically develops a wrinkle, such as a forehead, lips, eyelids, nasolabial fold, skin under an eye, or between the eye brows in order to prevent the development of a wrinkle.

The disclosed compositions can be administered to prevent scar formation not associated with a wound, such as a stretch mark, or scars resulting from acne, chicken pox, measles or other disease states. In certain embodiments, the disclosed compositions are administered to the area of skin expansion in order to prevent formation of such scars. In these embodiments, the composition can be administered to any region of a face, abdomen, breasts, arms, legs, buttocks, back, or any other area where the skin is susceptible to developing a scar.

The compositions can be administered prior to, concurrently with, and/or after the development of the skin blemish. For instance, the disclosed compositions can be administered prior to an incision, during a surgical procedure, and/or any time postoperatively, and then additionally administered after the procedure as the healing process occurs. In another example, the compositions can be administered during pregnancy to prevent stretch marks. Alternately, the compositions can be administered after the development of a blemish.

The compositions may be administered typically for 1 to 7 days , or for a period of time necessary to achieve the desired results, which may be several days to several months. The compositions can be administered once or several times (2, 3, 4, or more times) a day depending on the desired effect. In certain embodiments, the compositions can be administered every 1, 2, 3, 4, 5, 6, or 7 days. In another embodiment, the compositions can be administered one or more times every 1, 2, 3, or 4 weeks. The administration can be on a monthly or bi-monthly basis. Further, the compositions can be administered for 1, 2, 3, 6, 9, or 12 months or more. In certain embodiments, the compositions can be administered on an ongoing basis to maintain a desired result.

The disclosed compounds can be administered as part of a composition. As used herein, "formulation" and "composition" may be used interchangeably and refer to a combination of elements that is presented together for a given purpose. Such terms are well known to those of ordinary skill in the art.

As used herein, "carrier," "inert carrier," and "acceptable carrier" may be used interchangeably and refer to a carrier which may be combined with the presently disclosed compounds in order to provide a desired composition. Those of ordinary skill in the art will recognize a number of carriers that are well known for making specific pharmaceutical and/or cosmetic compositions. Desirably, the carrier is suitable for application to keratinous surfaces or other areas of the body. Upon application, acceptable carriers are substantially free of adverse reactions with skin and other keratinous surfaces. For example, the carriers may take the form of fatty or non-fatty creams, milky suspensions or emulsion-in-oil or oil-in-water types, lotions, gels or jellies, colloidal or non-colloidal aqueous or oily solutions, pastes, aerosols, soluble tablets or sticks. In accordance with one embodiment, the composition includes a dermatologically compatible vehicle or carrier. The vehicle which may be employed for preparing compositions may comprise, for example, aqueous solutions such as e.g., physiological salines, oil solutions or ointments. The vehicle furthermore may contain dermatologically compatible preservatives such as e.g., benzalkonium chloride, surfactants like e.g., polysorbate 80, liposomes or polymers, for example, methyl cellulose, polyvinyl alcohol, polyvinyl pyrrolidone and hyaluronic acid; these may be used for increasing the viscosity.

Examples of additional agents which can be included in the present compositions are anti-itch, anti-cellulite, anti-scarring, and anti-inflammatory agents, anesthetics, anti-irritants, vasoconstrictors, vasodilators, as well as agents to prevent/stop bleeding, and improve/remove pigmentation, moisturizers, desquamating agents, tensioning agents, anti-acne agents. Anti-itch agents can include methyl sulphonyl methane, sodium bicarbonate, calamine, allantoin, kaolin, peppermint, tea tree oil and combinations thereof. Anti-cellulite agents can include forskolin, xanthine compounds such as, but not limited to, caffeine, theophylline, theobromine, and aminophylline, and combinations thereof. Anesthetic agents can include lidocaine, benzocaine, butamben, dibucaine, oxybuprocaine, pramoxine, proparacaine, proxymetacaine, tetracaine, and combinations thereof. Anti-scarring agents can include IFN-.gamma., fluorouracil, poly(lactic-co-glycolic acid), methylated polyethylene glycol, polylactic acid, polyethylene glycol and combinations thereof. Anti-inflammatory agents can include dexamethasone, prednisolone, corticosterone, budesonide, estrogen, sulfasalazine, mesalamine and derivatives and combinations thereof. Additionally, active agents such as epinephrine, thymidine, cytidine, uridine, antiypyrin, aminocaproic acid, tranexamic acid, eucalyptol, allantoin, glycerin, and sodium selenite, can be included. Formulations can further comprise degradation inhibitors. Degradation inhibitors, include but are not limited to, glycosaminoglycans (e.g., heparin, heparin sulfate, dermatan sulfate, chrondroitin sulfate, o-sulfated HA, lnamarin, and amygdalin), antioxidants (e.g. ascorbic acid, melatonin, vitamin C, vitamin E), proteins (e.g., serum hyaluronidase inhibitor), and fatty acids (e.g. saturated C₁₀ to C₂₂ fatty acids). In certain embodiments, additional active agent is an antioxidant. In certain embodiments, the antioxidant comprises a vitamin C and/or a vitamin E such as d-alpha-tocopheryl polyethylene glycol 1000 succinate (TPGS).

The disclosed compositions are well suited for topical, subcutaneous, intradermal, subdermal, subcutaneous, and transdermal administration. Topical administration relates to the use of a composition applied to the surface of the skin at the site of a skin blemish for exertion of local action. Accordingly, such topical compositions include those pharmaceutical or cosmetic forms in which the composition is applied externally by direct contact with the skin surface to be treated, such as the face, neck, arms, legs, and/or torso. Conventional pharmaceutical or cosmetic forms for this purpose include ointments, liniments, creams, shampoos, lotions, pastes, jellies, sprays, aerosols, and the like, and may further be applied directly or in patches or impregnated dressings depending on blemish and skin region to be treated. The term "ointment" embraces formulations (including creams) having oleaginous, water-soluble and emulsion-type bases, e.g., petrolatum, lanolin, polyethylene glycols, as well as mixtures of these.

The compositions are appropriate for mesotherapy applications as well. Mesotherapy is a non-surgical cosmetic treatment technique involving intra-epidermal, intra-dermal, and/or subcutaneous injection of a composition. The compositions are administered in the form of small multiple droplets into the epidermis, dermo-epidermal junction, and/or the dermis.

In accordance with the disclosure, a pharmaceutical or cosmetic composition can optionally include one or more agents such as, without limitation, emulsifying agents, wetting agents, sweetening or flavoring agents, tonicity adjusters, preservatives, buffers antioxidants and flavonoids. Tonicity adjustors useful in a pharmaceutical composition of the present disclosure include, but are not limited to, salts such as sodium acetate, sodium chloride, potassium chloride, mannitol or glycerin and other pharmaceutically acceptable tonicity adjusters. Preservatives useful in the pharmaceutical compositions described herein include, without limitation, benzalkonium chloride, chlorobutanol, thimerosal, phenyl mercuric acetate, and phenyl mercuric nitrate. Various buffers and means for adjusting pH can be used to prepare a pharmaceutical composition, including but not limited to, acetate buffers, citrate buffers, phosphate buffers and borate buffers. Similarly, antioxidants useful in pharmaceutical compositions are well known in the art and include for example, sodium metabisulfite, sodium thiosulfate, acetylcysteine, butylated hydroxyanisole and butylated hydroxytoluene. Flavonoids are compounds found in plants that are well known to have diverse beneficial biochemical and antioxidant effects. Subcategories of flavonoids include: flavones, flavonols, flavanonse and flavanonols. Examples of flavonoids include: luteolin, apigenin, tangeritin, quercetin, kaempferol, myricetin, fisetin, isorhamnetin, pachypodol, rhamnazin, hesperetin, naringenin, eriodictyol, homoeriodictyol, taxifolin, dihydroquercetin, dihydrokaempferol, tannic acid, tannis, condensed tannis, and hydrolysable tannis. It is understood that these and other substances known in the art can be included in a pharmaceutical or cosmetic composition disclosed herein.

As used herein, the term "therapeutically effective amount" means the amount of the pharmaceutical or cosmetic composition that will elicit the biological, medical, or cosmetic response of a subject in need thereof that is being sought by the researcher, veterinarian, medical doctor or other clinician. In some embodiments, the subject in need thereof is a mammal. In certain embodiments, the mammal is human. Effective amounts of the compound may be determined by one of ordinary skill in the art but will vary depending on the compound employed, frequency of application and desired result, and will generally range from about 0.0000001% to about 50%, by weight, of the composition, preferably from about 0.001% to about 50%, by weight, of total composition, more preferably from about 0.001% to about 30%, by weight of the composition. In certain embodiments, the compound is about 0.004% by weight of the composition.

The compounds described herein may be administered at least in the minimum dose necessary to achieve the desired therapeutic effect. Generally, such doses will be in the range of about 1 mg/day to about 1000 mg/day; more preferably in the range of about 10 mg/day to about 500 mg/day. In another example embodiment, the compound or compounds may be present in a composition or formulation in a range of about 0.0001 mg/kg/day to about 100 mg/kg/day or about 0.01 mg/kg/day to about 100 mg/kg/day. However, the actual amount of the compound to be administered in any given case will be determined by a physician taking into account the relevant circumstances, such as the age and weight of a patient, patient's general physical condition, severity of the skin blemish, and route of administration. In some instances, dosing is evaluated on a case-by-case basis.

Additionally, compositions may be designed to delay release of the compound over a given period of time, or to carefully control the amount of compound released at a given time during the course of treatment.

The pH of the disclosed compositions can be about 3 to about 8.0, or about 6.5 to about 7.5. In certain embodiments, the pH of the formulation is about 7.0 to about 7.4 or about 7.1 to about 7.3.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

Any reference made to patents and printed publications throughout this specification is individually incorporated herein by reference in its entirety.

It is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The present invention can be summarized by reference to the following embodiments (embs.):
Emb. 1. A method of treating a skin blemish comprising administering a composition comprising a therapeutically effective amount of a compound having a structure: 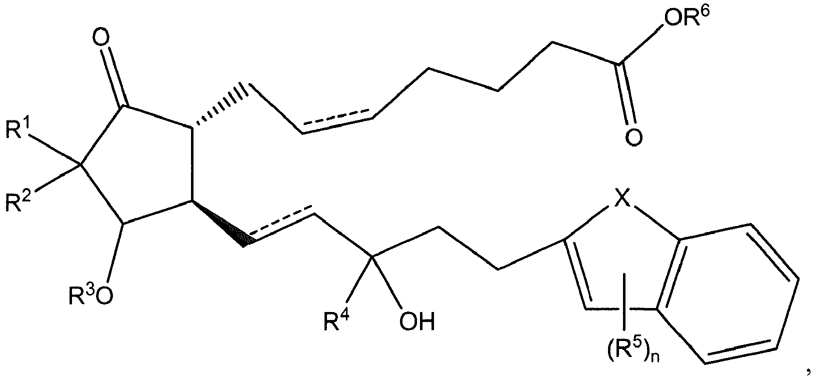 wherein each dashed line represents the presence or absence of a double bond; R¹, R¹, R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
   wherein said administration treats said skin blemish.
Emb. 2. The method of emb. 1, wherein R⁴ is H, R³ is H, and X is S.
Emb. 3. The method of emb. 1, wherein R¹ and R² are CH³.
Emb. 4. The method of emb. 1, wherein R⁵ is Cl.
Emb. 5. The method of emb. 1, wherein the compound is : 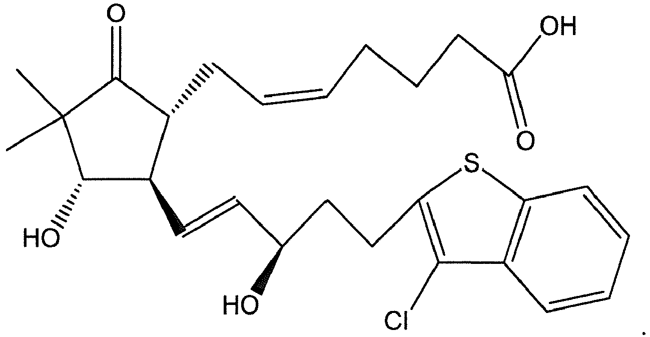
Emb. 6. The method of emb. 1, wherein the skin blemish is a flesh wound, scar, or wrinkle.
Emb. 7. The method of emb. 1, wherein the composition is administered subcutaneous, subdermal or transdermal, intradermally or topically.
Emb. 8. The method of emb. 6, wherein the administration reduces formation of a scar type selected from the group consisting of hypertrophic scar, recessed scar, stretch mark, and a combination thereof.
Emb. 9. The method of emb. 6, wherein the skin blemish is a wrinkle.
Emb. 10. The method of emb. 1, wherein the composition is administered to a region selected from the group consisting of a face, neck, arms, torso, back, legs, and a combination thereof.
Emb. 11. The method of emb. 1, wherein the composition is administered at a time selected from the group consisting of prior to surgical incision, during surgery, postoperatively, and a combination thereof.
Emb. 12. The method of emb. 1, wherein said administration minimizes scar formation.
Emb. 13. The method of emb. 1, wherein said administration prevents scar formation.
Emb. 14. The method of emb. 1, wherein said administration prevents wrinkle formation.
Emb. 15. The method of emb. 1, wherein said administration reduces the appearance of an existing wrinkle.
Emb. 16. The method of emb. 9, wherein the wrinkle selected from the group consisting of a brow furrow, crow's feet, nasolabial fold, a line under the eye, a crease between the eye brows, and a combination thereof.
Emb. 17. The method of emb. 6, wherein a cause of said flesh wound is selected from the group consisting of an incision, a laceration, a thermal burn, a chemical burn, an abrasion, a puncture wound, and a combination thereof.
Emb. 18. A method is provided for treating a flesh wound that comprises administering a composition comprising a therapeutically effective amount of a compound having a structure: 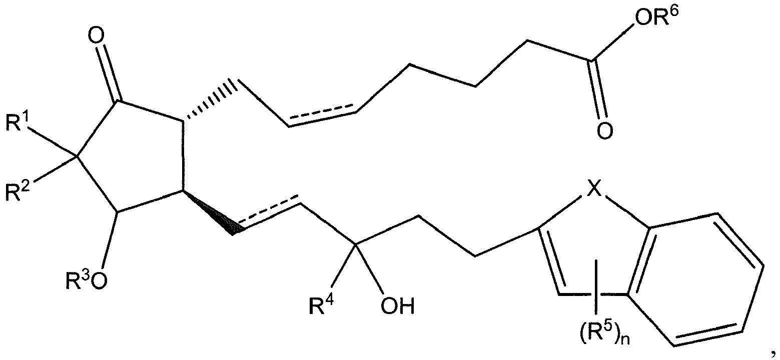 wherein each dashed line represents the presence or absence of a double bond; R¹, R², R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
   wherein said wound heals more normally than without administration of said composition.
Emb. 19. The method of emb. 18, wherein the compound is Compound 1: 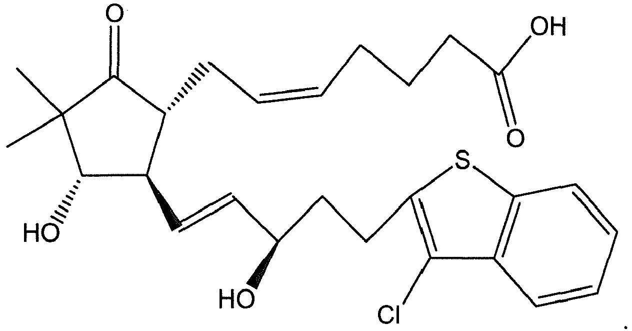
Emb. 20. A method of reducing the appearance of a wrinkle comprising administering to said wrinkle a composition comprising a therapeutically effective amount of a compound having a structure: 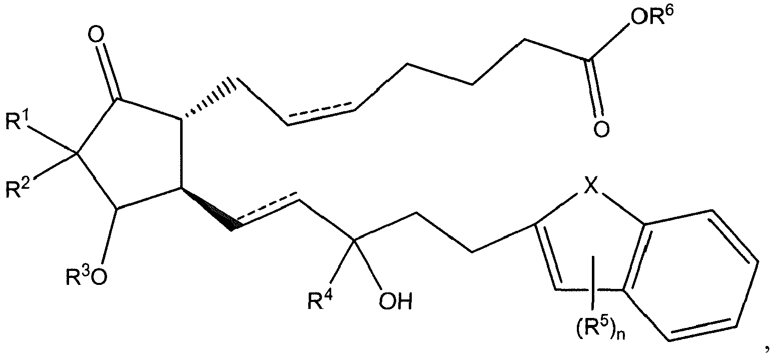 wherein each dashed line represents the presence or absence of a double bond; R¹, R¹, R³ and R⁴ are each independently selected from H and C₁-C₆ linear alkyl; R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
   wherein the appearance of said wrinkle is diminished.
Emb. 21. The method of emb. 20, wherein the compound is: 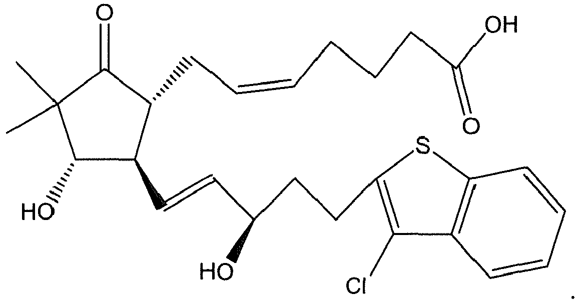
Emb. 22. The method of emb. 20, wherein said composition is administered topically.
Emb. 23. A method of treating a skin blemish comprising administering to a subject in need thereof a composition comprising a therapeutically effective amount of at least one EP4 agonist having the structure: 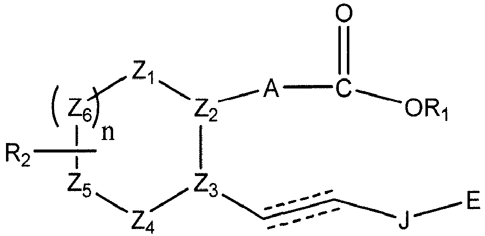 wherein:
   each of Z₁ to Z₆ is independently C, N, O, or S;
   A is -(CH₂)₆-, or *cis* -CH₂CH=CH-(CH₂)₃-, wherein 1 or 2 carbons may be substituted with S or O; or
   A is -(CH₂)ₘ-Ar-(CH₂)ₒ- wherein Ar is arylene or heteroarylene, the sum of m and o is from 1 to 4, and wherein one CH₂ may be substituted with S or O;
   R₁ is H, alkyl, cycloalkyl, oxyalkyl, hydroxyalkyl, alkenyl, oxyalkenyl, or hydroxyalkenyl;
   R₂ is alkyl, hydroxyl, halide, or oxo;
   J is alkylene, cycloalkylene, oxyalkylene, hydroxyalkylene, fluoroalkylene, or difluoroalkylene;
   E is C₁₋₁₂ alkyl, R₃, or -Y-R₃ wherein Y is CH₂, S, or O, and R₃ is aryl or heteroaryl;
   n is 0 or 1;
   and wherein a dashed line represents the presence or absence of a bond.
Emb. 24. A method of treating a skin blemish comprising administering to a subject in need thereof a composition comprising a therapeutically effective amount of at least one EP4 agonist having the structure: 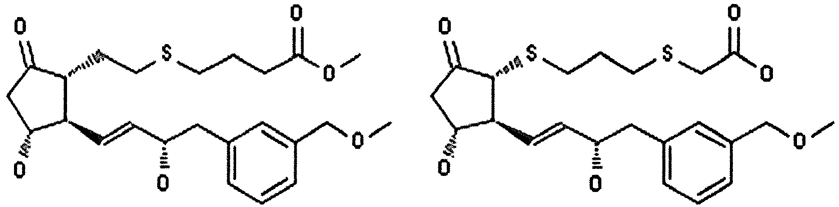 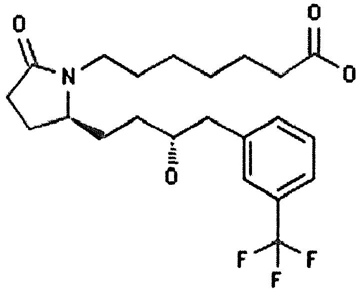 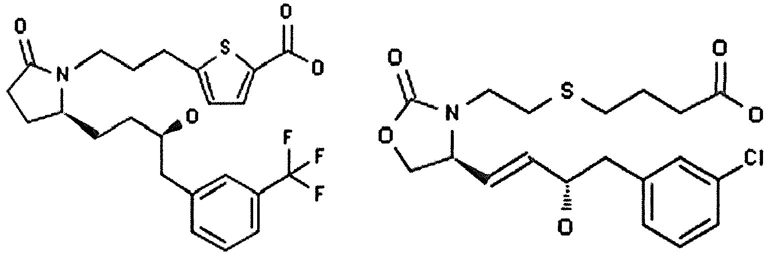 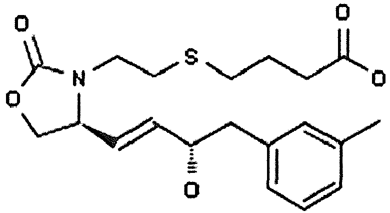 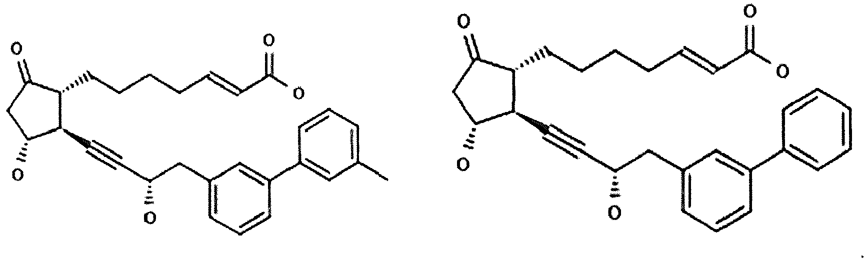
Emb. 25. A method of treating a skin blemish comprising administering to a subject in need thereof a composition comprising a therapeutically effective amount of at least one EP4 agonist having the structure: 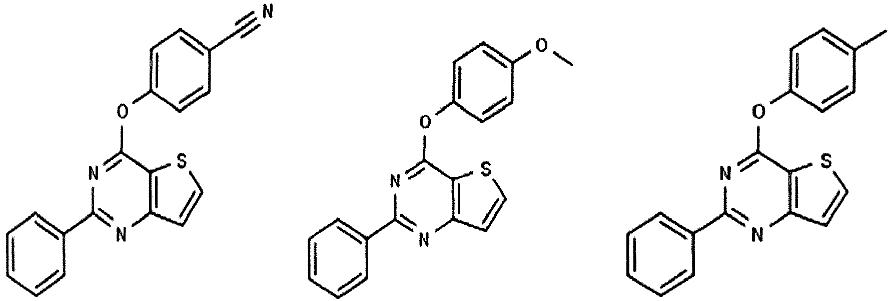 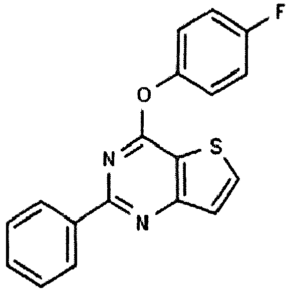 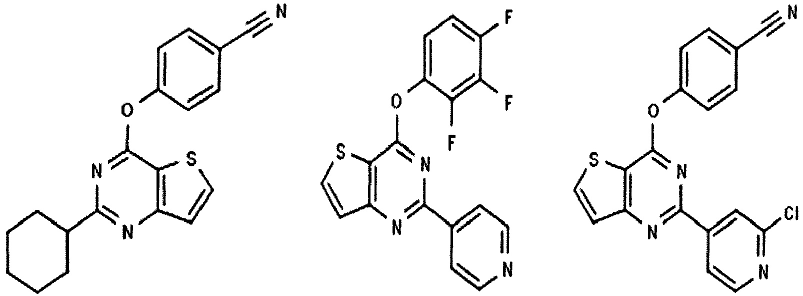 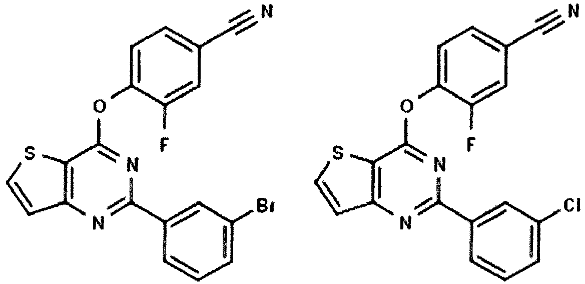 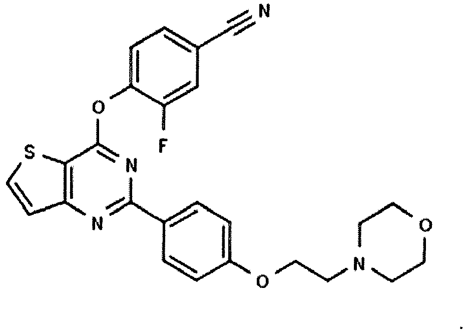
Emb. 26. A method of treating a skin blemish comprising administering to a subject in need thereof a composition comprising a therapeutically effective amount of at least one EP4 agonist having the structure:

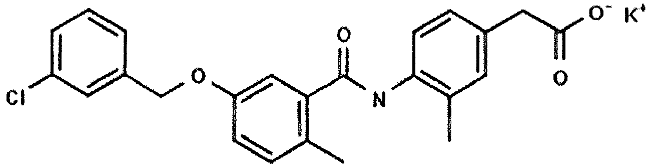

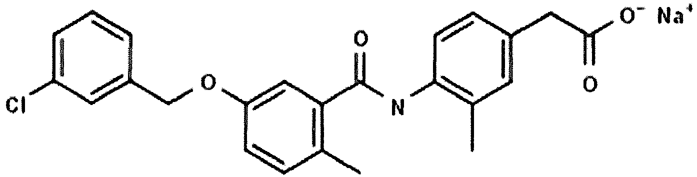

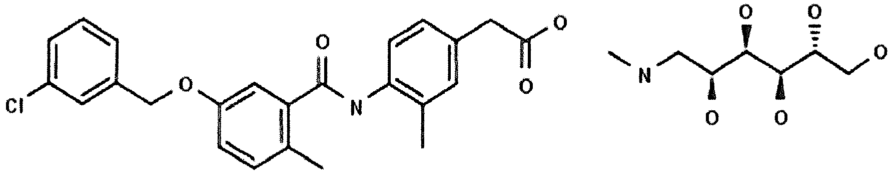

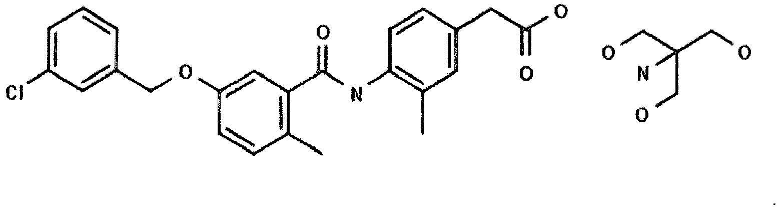

## Claims

1. Compound for use in the treatment of skin blemish, said compound having the structure: 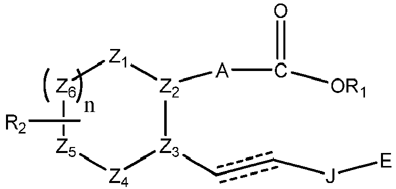 wherein:
each of Z₁ to Z₆ is independently C, N, O, or S;
A is -(CH₂)₆-, or *cis* -CH₂CH=CH-(CH₂)₃-, wherein 1 or 2 carbons may be substituted with O; or
A is -(CH₂)ₘ-Ar-(CH₂)ₒ- wherein Ar is arylene or heteroarylene, the sum of m and o is from 1 to 4, and wherein one CH₂ may be substituted with S or O;
R₁ is H, alkyl, cycloalkyl, oxyalkyl, hydroxyalkyl, alkenyl, oxyalkenyl, or hydroxyalkenyl;
R₂ is alkyl, hydroxyl, halide, or oxo;
J is alkylene, cycloalkylene, oxyalkylene, hydroxyalkylene, fluoroalkylene, or difluoroalkylene;
E is C₁₋₁₂ alkyl, R₃, or -Y-R₃ wherein Y is CH₂, S, or O, and R₃ is aryl or heteroaryl;
n is 0 or 1;
and wherein a dashed line represents the presence or absence of a bond.

2. Compound for use according to claim 1, said compound having the structure: 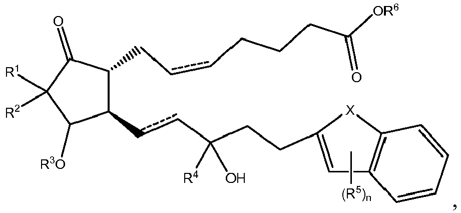
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

3. The compound for use of claim 2, wherein R⁴ is H, R³ is H, and X is S.

4. The compound for use of claim 2, wherein R¹ and R² are CH₃.

5. The compound for use of claim 2, wherein R⁵ is Cl.

6. The compound for use of claim 2, wherein the compound is

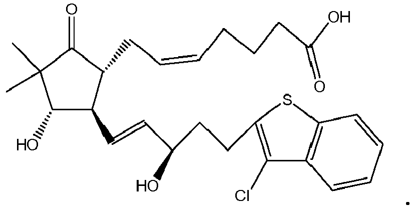

7. The compound for use of any one of claims 1 to 6, wherein the skin blemish is a flesh wound, scar, or wrinkle.

8. The compound for use of claim 7, wherein the wrinkle selected from the group consisting of a brow furrow, crow's feet, nasolabial fold, a line under the eye, a crease between the eye brows, and a combination thereof.

9. The compound for use of claim 7, wherein a cause of said flesh wound is selected from the group consisting of an incision, a laceration, a thermal burn, a chemical burn, an abrasion, a puncture wound, and a combination thereof.

10. The compound for use of claim 1, said compound having the structure:
